# EUROPEAN PATENT APPLICATION

(11) **EP 4 800 100 A1**
(43) Date of publication of application: **02.09.2026**
(21) Application number: 24881733.0
(22) Date of filing: 25.10.2024
(51) Int. Cl.: C12N 7/01, A61P 35/00, A61K 35/761

(54) **ONCOLYTIC VIRUS AND USE THEREOF**

(30) Priority: 27.10.2023 CN 202311414442
(71) Applicant: Shanghai Yuansong Biotechnology Co. Ltd., Shanghai 201499 (CN)
(72) Inventor: ZHANG, Kangjian, Shanghai 201499 (CN); FANG, Xianlong, Shanghai 201499 (CN); CHEN, Ruoyu, Shanghai 201499 (CN); YANG, Yuanyuan, Shanghai 201499 (CN); LIU, Yanqiu, Shanghai 201499 (CN); SUN, Aixi, Shanghai 201499 (CN); LU, Lingyu, Shanghai 201499 (CN)
(74) Representative: Witte, Weller & Partner Patentanwälte mbB
(86) International application number: PCT/CN2024/127294
(87) International publication number: WO 2025/087372

(57) **Abstract**

Provided are an oncolytic virus formulation for nebulizer therapy of lung cancer, and a method for nebulizer therapy of lung cancer by using an oncolytic virus, wherein the oncolytic virus is an interferon-expressing oncolytic virus.

## Description

### CROSS REFERENCE TO RELATED APPLICATIONS

The present application claims priority to Chinese Patent Application No. 202311414442.6 filed on October 27, 2023.

All publications, patents, and patent applications cited in the present application are incorporated herein by reference as if each publication, patent, and patent application were specifically and individually set forth and incorporated herein by reference in their entirety.

### FIELD

The present disclosure belongs to the technical field of medicine, and relates to an oncolytic virus formulation for nebulization therapy of lung cancer, and further relates to a method for treating lung cancer by nebulization therapy using an oncolytic virus.

### BACKGROUND

Oncolytic viruses (OVs) are a class of viruses that are natural or genetically engineered, can selectively replicate in tumor tissue and thereby infect and kill tumor cells or cause tumor cell lysis, and have no killing effect on normal tissue. Owing to their favorable tumor targeting, unique anti-tumor mechanisms and the like, they have become one of the research hotspots in tumor treatment strategies in recent years. Compared with conventional immunotherapy, oncolytic viruses have advantages such as favorable targeting, mild adverse reactions, multiple tumor-killing pathways, and low susceptibility to drug resistance. Oncolytic viruses have numerous sources, including adenovirus, herpes simplex virus, vaccinia virus, reovirus, measles virus, and the like. Among them, oncolytic adenovirus has many advantages, including high safety, many indications, easy production and purification, and biological stability, and therefore oncolytic adenovirus is now used very widely.

Aerosolized administration therapy mainly refers to aerosol inhalation therapy, which has a history of several thousand years to date. In ancient times, diseases were mainly treated by oral or nasal inhalation in forms such as smoke, vapor, medicated pillows, and sachets. Aerosol inhalation therapy is a primary method for treating respiratory system diseases and has currently been widely used clinically. The lung has characteristics of a relatively thin alveolar cell membrane, a relatively rich capillary network, and a relatively slow clearance rate in deep lung. Compared with other administration modes, aerosol inhalation administration treatment has characteristics of a small drug dose, facilitated inhalation, and a large absorption surface area, can rapidly exert an effect at a target site, reduces adverse effects, and is relatively convenient to use.

At present, administration modes for oncolytic adenovirus are limited to intratumoral administration. For patients having tumor lesions in the lung, percutaneous lung puncture administration has relatively high risks and is not suitable for periodic repeated administration. Moreover, tumor patients with lung metastasis often have multiple lesions in the lung, making treatment by percutaneous lung puncture administration even less feasible. Therefore, developing an aerosolized administration mode for oncolytic adenovirus will bypass the limitations of intratumoral administration, broaden indications, improve operational convenience, and improve the patient survival rate.

### SUMMARY

Through research, the inventors unexpectedly found that nebulization therapy of lung cancer with an oncolytic virus has an unexpected therapeutic effect, and completed the present disclosure on this basis.

In a first aspect, the present disclosure provides a use of an oncolytic virus in the manufacture of an aerosol drug for treating lung cancer, wherein the oncolytic virus is an interferon-expressing oncolytic virus.

In some embodiments, the interferon is interferon α, interferon β, interferon γ, or consensus interferon. Preferably, the interferon is interferon α; more preferably, the interferon is consensus interferon; and further preferably, the interferon is encoded by a nucleotide sequence of SEQ ID NO: 1, SEQ ID NO: 2, or SEQ ID NO: 3.

ATGGCCCTGTCCTTCAGCCTGCTGATGGCCGTGCTGGTGCTGAGCTACAAGTCC
ATCTGCTCCCTGGGCATGTGTGATCTGCCTCAGACACACTCCCTGGGCAATAGAAGGG
CCCTGATCCTGCTGGCCCAGATGAGAAGGATCAGCCCCTTCTCCTGCCTGAAGGATAG

ACACGATTTTGGCTTCCCTCAGGAGGAGTTCGACGGCAATCAGTTTCAGAAGGCCCA
GGCCATCTCCGTGCTGCACGAGATGATCCAGCAGACCTTTAACCTGTTCTCCACAAAG
GACTCCAGCGCCGCCTGGGACGAGTCCCTGCTGGAGAAGTTTTACACAGAGCTGTAC
CAGCAGCTGAACGATCTGGAGGCCTGCGTGATCCAGGAGGTGGGCGTGGAGGAGAC
CCCCCTGATGAATGTGGATTCCATCCTGGCCGTGAAGAAGTACTTTCAGAGAATCACC
CTGTACCTGACCGAGAAGAAGTACAGCCCTTGTGCCTGGGAGGTGGTGAGAGCCGAG
ATCATGAGATCCTTTTCCCTGAGCACAAACCTGCAGGAGAGGCTGAGAAGGAAGGAG
TGA (SEQ ID NO: 1)

ATGGCCCTGTCCTTCTCCCTGCTGATGGCCGTGCTGGTGCTGAGCTACAAGTCC
ATCTGCTCCCTGGGCATGTGCGACCTGCCTCAGACACACTCCCTGGGCAATAGGAGA
GCCCTGATCCTGCTGGCCCAGATGAGGAGGATCTCCCCTTTTAGCTGCCTGAAGGATA
GACACGATTTCGGCTTCCCTCAGGAGGAGTTCGATGGCAATCAGTTCCAGAAGGCCC
AGGCCATCAGCGTGCTGCACGAGATGATCCAGCAGACCTTCAATCTGTTTAGCACCAA
GGACTCCAGCGCCGCCTGGGACGAGTCCCTGCTGGAGAAGTTCTACACCGAGCTGTA
CCAGCAGCTGAACGACCTGGAGGCCTGCGTGATCCAGGAGGTGGGCGTGGAGGAGA
CCCCTCTGATGAATGTGGATAGCATCCTGGCCGTGAAGAAGTACTTTCAGAGAATCAC
ACTGTACCTGACAGAGAAGAAGTACAGCCCCTGCGCCTGGGAGGTGGTGAGGGCTG
AGATCATGAGGAGCTTTTCCCTGTCCACAAACCTGCAGGAGAGGCTGAGAAGGAAGG
AGTGA (SEQ ID NO: 2)

ATGGCCCTGTCCTTTTCTTTACTGATGGCCGTGCTGGTGCTCAGCTACAAATCCA
TCTGTTCTCTGGGCATGTGCGACCTGCCGCAGACCCACTCCCTGGGTAACCGTCGTGC
TCTGATCCTGCTGGCTCAGATGCGTCGTATCTCCCCGTTCTCCTGCCTGAAAGACCGTC
ACGACTTCGGTTTCCCGCAGGAAGAATTCGACGGTAACCAGTTCCAGAAAGCTCAGG
CTATCTCCGTTCTGCACGAAATGATCCAGCAGACCTTCAACCTGTTCTCCACCAAAGA
CTCCTCCGCTGCTTGGGACGAATCCCTGCTGGAAAAATTCTACACCGAACTGTACCAG
CAGCTGAACGACCTGGAAGCTTGCGTTATCCAGGAAGTTGGTGTTGAAGAAACCCCG
CTGATGAACGTTGACTCCATCCTGGCTGTTAAAAAATACTTCCAGCGTATCACCCTGTA
CCTGACCGAAAAAAAATACTCCCCGTGCGCTTGGGAAGTTGTTCGTGCTGAAATCATG
CGTTCCTTCTCCCTGTCCACCAACCTGCAGGAACGTCTGCGTCGTAAAGAATAA (SEQ
ID NO: 3)

In some embodiments, the oncolytic virus is an attenuated wild-type virus strain, such as a reovirus and a Newcastle disease virus; or the oncolytic virus is a genetically engineered virus, such as an engineered adenovirus, a herpes simplex virus, a vaccinia virus, and a measles virus. Preferably, the oncolytic virus is an oncolytic adenovirus.

In some embodiments, the aerosol drug is administered by the following means: a metered dose inhaler, a dry powder inhaler, and/or a nebulizer, preferably a nebulizer. In other embodiments, the nebulizer is selected from the group consisting of a pneumatic jet nebulizer, an ultrasonic nebulizer, and a mesh nebulizer.

In some embodiments, the lung cancer is small cell lung cancer, lung squamous cell carcinoma, lung adenocarcinoma, large cell lung cancer, and/or metastatic lung tumor.

In a second aspect, the present disclosure provides an oncolytic virus for treating lung cancer, wherein the oncolytic virus is administered in a dosage form of aerosol, and the oncolytic virus is as described in the first aspect.

In a third aspect, the present disclosure provides a method for treating lung cancer, comprising administering an oncolytic virus to a subject in need thereof via nebulization, wherein the oncolytic virus is as described in the first aspect.

In some embodiments, the method further comprises administering the oncolytic virus to the subject by intratumoral injection and/or intraperitoneal injection.

In other embodiments, the method further comprises administering an antitumor drug to the subject, wherein the antitumor drug is selected from the group consisting of a chemotherapeutic drug, such as paclitaxel, cisplatin, carboplatin, pemetrexed, docetaxel, gemcitabine, and irinotecan, a targeting therapeutic drug, such as gefitinib, osimertinib, erlotinib, afatinib, and lapatinib, and an immunotherapeutic drug, such as bevacizumab, nimotuzumab, sintilimab, camrelizumab, nivolumab, pembrolizumab, atezolizumab, durvalumab, and ipilimumab.

In other embodiments, the method further comprises performing radiotherapy on the subject.

In a fourth aspect, the present disclosure provides an aerosol pharmaceutical composition, comprising an oncolytic virus, wherein the oncolytic virus is as described in the first aspect, and the aerosol pharmaceutical composition is in a liquid form or an aerosol form.

In a fifth aspect, the present disclosure provides an aerosol kit, comprising a nebulization device and a drug, wherein the drug comprises an oncolytic virus, the drug is in a liquid form or an aerosol form, and the oncolytic virus is as described in the first aspect. In some embodiments, the nebulization device is selected from the group consisting of a metered dose inhaler, a dry powder inhaler, and a nebulizer, preferably a nebulizer, such as a pneumatic jet nebulizer, an ultrasonic nebulizer, and a mesh nebulizer.

### BRIEF DESCRIPTION OF DRAWINGS

In order to more clearly describe the technical solutions in the embodiments of the present disclosure or in the prior art, the drawings used in the description of the embodiments or the prior art are briefly introduced below. Obviously, the drawings in the following description are merely some embodiments of the present disclosure, and a person of ordinary skill in the art may obtain other drawings based on these drawings without any creative effort.
FIG. 1 shows detection results of mice in Example 2. FIG. 1A shows *in vivo* fluorescence imaging images of mice in each group, and FIG. 1B shows statistical analysis results of the signals.
FIG. 2 shows survival curves of mice in the control group (Vehicle) and the nebulization treatment group (YSCH-01) in Example 2.
FIG. 3 shows administration time and administration mode for patient 1 in Example 3.
FIG. 4 shows detection results of immune cell population proportions before and after oncolytic virus treatment.

### DETAILED DESCRIPTION

The technical solution will be clearly and completely described below in conjunction with examples of the present disclosure. Obviously, the described examples are merely illustrative, and are not an exhaustive listing of all embodiments of the present disclosure. Based on the examples of the present disclosure, all other embodiments obtained by a person of ordinary skill in the art without creative effort fall within the scope of protection of the present disclosure.

Unless otherwise specified, terms used herein have the ordinary meanings understood by a person of ordinary skill in the technical field to which they belong.

The term "oncolytic virus" refers to a virus capable of selectively replicating in cancer or hyperproliferative cells, thereby slowing their growth or causing their death, while having no effect or little effect on normal cells. Exemplary oncolytic viruses include vesicular stomatitis virus (VSV), Newcastle disease virus (NDV), herpes simplex virus (HSV), reovirus, measles virus, retrovirus, influenza virus, Sindbis virus, vaccinia virus, adenovirus, and the like.

In the present disclosure, the unit of viral dose is vp (viral particle), which represents the number of viral particles contained in 1 ml of a virus solution, and is the particle titer of the virus, typically expressed as vp/ml. In some specific embodiments, it may also be expressed as vp/cell (viral particle per cell) or in another suitable manner of expression.

The terms "treatment" and "method for treatment" include therapeutic treatment and prophylactic treatment. Those in need of treatment may include those already suffering from a particular medical disease and those that may ultimately suffer from the disease. Treating a tumor includes eliminating or inhibiting tumor growth, inhibiting cancer metastasis, and eliminating residual tumor after and/or during surgery or other treatment.

The term "subject" includes any human or non-human animal. The term "non-human animal" includes all vertebrates, for example mammals and non-mammals, such as non-human primates, sheep, dogs, cats, horses, cattle, chickens, amphibians, reptiles, and the like. Unless otherwise specified, the terms "patient" and "subject" may be used interchangeably.

The term "administering" or "administration" means providing a substance, such as an oncolytic virus, to a subject in a pharmacologically usable manner.

The dose of a pharmaceutical composition provided to a subject refers to a dose sufficient to show a benefit to the subject to which it is administered, and may also be referred to herein as a "pharmaceutically effective amount" or an "effective amount." The actual amount administered, as well as the rate and time course of administration, depend on the individual condition and severity of the subject being treated. Prescription of treatment, for example determination of dosage and the like, is ultimately the responsibility of physicians and other medical doctors and depends on their decisions, usually taking into account the disease being treated, the individual condition of the patient, the delivery site, the method of administration, and other factors known to doctors.

The term "aerosol drug" or "aerosolised drug" and "aerosol" may be used interchangeably herein, and refer to a drug dosage form capable of being aerosolized, which usually is taken by a subject via inhalation so that the drug is delivered to the distal lung or deposited on the proximal airway, thereby producing a therapeutic effect. Before being aerosolized, the drug may be in a solid form, for example dry powders, or may be in a liquid form.

The terms "aerosol therapy" and "nebulization therapy" may be used interchangeably herein, and refer to administering a drug by nebulization, namely administering the drug to a subject after the drug is nebulized by a nebulization device.

The term "consensus interferon" has the meaning known to those skilled in the art, and is an artificially recombinant interferon developed by performing multiple sequence alignment of a plurality of natural human interferon α subtypes and then assigning, at each position, an amino acid having a relatively high frequency of occurrence, and its amino acid sequence is as shown in SEQ ID NO: 4.

As used herein, unless otherwise expressly indicated, the absence of an article before a noun term or modification of the term by "the" indicates that the term may be one or more.

The term "about" or "approximately" generally indicates within an error range of a particular value determined by a person of ordinary skill, which depends in part on the manner of measurement, that is, is limited by the measurement system. For example, according to practice in a particular field, "about" may refer to one or more standard deviations. In particular, "about" may refer to values within a range of 20%, 10%, 5%, or 1% deviation from a given value.

The technical solution will be clearly and completely described below in conjunction with examples of the present disclosure. Obviously, the described examples are merely illustrative, and are not an exhaustive listing of all embodiments of the present disclosure. Based on the examples of the present disclosure, all other embodiments obtained by a person of ordinary skill in the art without creative effort fall within the scope of protection of the present disclosure.

### Examples

### Example 1: Formulation and Validation of an Oncolytic Virus Formulation for Nebulized Administration

The oncolytic virus used in the present disclosure has been disclosed in CN111363726A. The oncolytic virus in Example 1 carries interferon encoded by SEQ ID NO: 1, which is abbreviated herein as YSCH-01, and the preparation method may be found in patent application publication CN111363726A.

### 1. Formulation

The formulation buffer (Tris, MgCl₂·H₂O, Sucrose, NaCl, absolute ethanol, Tween 80, and HCl) was prepared in a volume of 10 L. The oncolytic virus sample or the cryopreserved oncolytic virus sample was added to the buffer to prepare an oncolytic virus formulation.

### 2. Adenovirus Infectious Titer Assay

(1) Plating 293 cells: 293 cells in good growth condition were taken and passaged after trypsin digestion. The cells were resuspended in complete culture medium and counted using a cell counting chamber, the cell density was adjusted to 1×10⁵ cells/ml, and 100 µl of cells was seeded into each well of a 96-well plate (1×10⁴ cells/well), followed by overnight incubation (18 to 22 h) in an incubator at 37°C and 5% CO₂.
(2) Virus dilution: sterile 5 ml EP tubes were taken and numbered in advance, the oncolytic virus formulation was diluted 10-fold with serum-free DMEM medium (an appropriate dilution factor was selected according to the specific situation), and then 2-fold diluted for 8 titer levels to serve as test samples. The specific dilution process is shown in Table 2:

**Table 2**

| EP tube number | Dilution | Virus volume (µl) | Diluent volume (µl) |
|---|---|---|---|
| 1 | 10¹ | 100 µl sample stock solution | 900 |
| 2 | 10² | 100 µl of 1 | 900 |
| 3 | 10³ | 100 µl of 2 | 900 |
| 4 | 10⁴ | 100 µl of 3 | 900 |
| 5 | 10⁵ | 100 µl of 4 | 900 |
| 6 | 10⁶ | 100 µl of 5 | 900 |
| 7 | 2×10⁶ | 2400 µl of 7 | 2400 |
| 8 | 4×10⁶ | 2400 µl of 8 | 2400 |
| 9 | 8×10⁶ | 2400 µl of 9 | 2400 |
| 10 | 1.6×10⁷ | 2400 µl of 10 | 2400 |
| 11 | 3.2×10⁷ | 2400 µl of 11 | 2400 |
| 12 | 6.4×10⁷ | 2400 µl of 12 | 2400 |
| 13 | 1.28×10⁸ | 2400 µl of 13 | 2400 |
| 14 | 2.56×10⁸ | 2400 µl of 14 | 2400 |

(3) Virus infection: the 96-well plate was taken out, and the old medium in the plate was gently aspirated with a multichannel pipette. Test samples of at least the last 8 dilutions, from low concentration to high concentration, were then sequentially added to the 96-well plate at 200 µl/well, with 10 replicate wells inoculated for each dilution; serum-free DMEM culture medium was added to the first column and the last column as a negative control at 200 µl/well. The plate was cultured for 60 min under conditions of 37°C and 5% CO₂.
(4) After virus infection for 60 min, the culture medium in each well was carefully aspirated, and 200 µl of DMEM culture medium containing 5% FBS was added. The operation was performed from low-concentration wells to high-concentration wells, and a new pipette tip was replaced for each dilution. Continuous culture was performed for 10 days under conditions of 37°C and 5% CO₂.
(5) Observation of experimental results: from Day 8 to Day 10 after inoculation of the test samples, the occurrence of cytopathic cells in each row was observed daily until the cytopathic cells no longer increased, and the number of wells in which cytopathic effect occurred was counted.
(6) Result calculation

Dilutions at which the ratio of wells with cytopathic effect was 20% to 80% (i.e., the number of wells with cytopathic cells at the same dilution was 2 to 8) were substituted into the following formula for calculation. Infectious titer per milliliter (IU/ml):
$V = - \left[\left(ln\left(1-Pw/n\right)\right)\times D\right] / \left(Aw\times Cw\times I\times {t}^{1 / 2}\right)$

In the formula, Pw/n is the positive well ratio; D is the dilution; Aw is the area of each well of the 96-well plate (cm²), 0.33 cm²; Cw is the cell confluence before virus inoculation, 80%; I is the diffusion coefficient, 2.38×10⁻⁴ cm/s^{1/2}; and t is the virus infection time (s), 3600 s. The calculated results were averaged, which was the viral titer assay result of the test sample.

### 3. Adenovirus Particle Count Assay

(1) The liquid chromatography equipment used was Shimadzu; the chromatography column used was a GE QXL chromatography column.
(2) The mobile phases used included: mobile phase A solution contained 50 mmol/L Tris-HCl and 300 mmol/L NaCl, pH 8.0; mobile phase B solution contained 50 mmol/L Tris-HCl and 1 mol/L NaC1, pH 8.0.
(3) The chromatographic analysis program is shown in Table 3:

**Table 3**

| Time (min) | Unit | Processing command | Value (%) |
|---|---|---|---|
| 0.01 | Pump | B.Conc | 20 |
| 5.00 | Pump | B.Conc | 20 |
| 15.00 | Pump | B.Conc | 100 |
| 20.00 | Pump | B.Conc | 100 |
| 23.01 | Pump | B.Conc | 20 |
| 28.00 | Pump | B.Conc | 20 |
| 28.01 | Controller | Stop | |

(4) The sample was directly injected and analyzed according to the chromatographic program. A standard curve was prepared from the reference standard, with the number of viral particles as the X-axis and the main peak area as the Y-axis, and linear fitting was performed. The main peak area value of the test sample was substituted into the fitted formula for calculation.

### 4. Nebulization of the Oncolytic Virus Formulation

An Aerogen vibrating mesh nebulizer Aerogen Solo (Cat. No.: AG-AS3200) was used to nebulize the oncolytic virus formulation, and the nebulized sample was collected and tested for virus concentration by the same method.

5. The results are shown in Table 4:

**Table 4**

| Oncolytic virus formulation | Content before nebulization | Content after nebulization | Infectious titer before nebulization | Infectious titer after nebulization |
|---|---|---|---|---|
| Viral content (number of viral particles) | 2.84E+11 VP/ml | 5.06E+10 VP/ml | 6.38E+09 IU/ml | 6.04E+08 IU/ml |

The results of the effect of nebulization on the content of the formulation drug solution (number of viral particles) showed that the nebulized formulation still had a very high viral infectious titer, indicating that the oncolytic virus formulation of the present disclosure had low nebulization loss and a high recovery rate, which was beneficial for ensuring clinical efficacy.

### Example 2: Treating Orthotopic Lung Tumors in Nude Mice by YSCH-01 Nebulized Administration

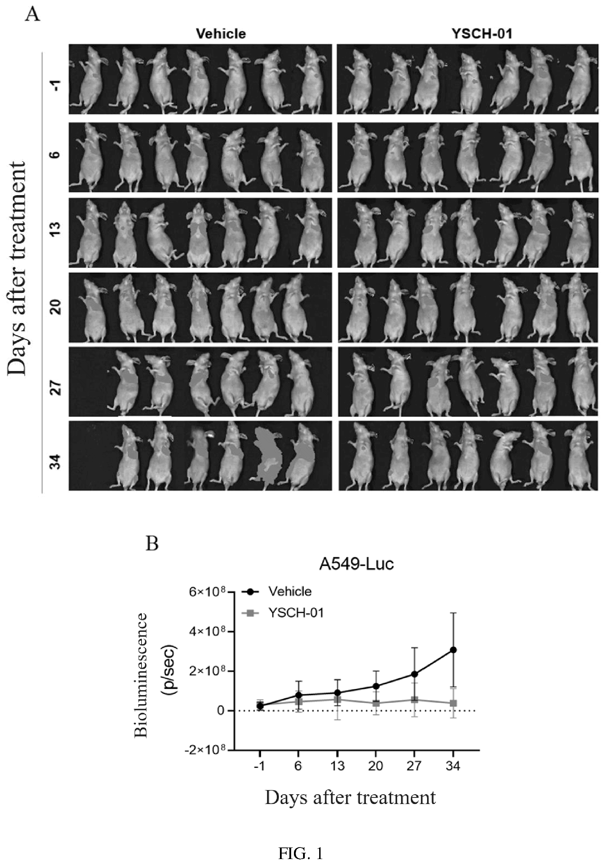

Lentivirus pLenti-CBh-3xFLAG-Luc2-tCMV-tdTomato-F2A-Puro-WPRE (OBiO, H7657) and A549 cells were used to construct an A549-luc cell line stably expressing luciferase. A549-Luc cells were resuspended in complete culture medium, and then 5×10⁶ cells (100 µl) were inoculated into 5- to 6-week-old BALB/c mice via the tail vein. On Day 9 after cell inoculation, the mice were intraperitoneally injected with potassium luciferin, and in vivo imaging signal detection in mice was performed 10 min later. The mice were grouped according to signal intensity, with 7 mice in each group, so that the average fluorescence intensity of the mice in each group was made as consistent as possible. The grouping information was: (A) Vehicle group and (B) YSCH-01 group. At this time (Day 9 after cell inoculation), not all mice had displayed obvious fluorescence signals; however, as the experimental time progressed, all mice in the Vehicle group (control group) exhibited fluorescence, demonstrating that modeling was successful in all mice. The YSCH-01 group received treatment, so not all mice exhibited fluorescence as the experimental time progressed. On Day 10 after cell inoculation, intervention was started, wherein (A) the Vehicle group was intervened with the virus vehicle and served as a negative control group, and (B) the YSCH-01 group was the treatment group. The administration mode was nebulized administration, specifically: a metered-dose administration device was placed in the trachea and was capable of delivering a metered amount of aerosol into the trachea and lungs of rats and mice; the diameter of the nebulized drug particles was 10 to 30 µm; the dose administered to the YSCH-01 group was 1.0×10¹⁰ vp/mouse/time; and the administration volume of both groups was 50 µl. Administration was performed once per week for a total of 3 times (QW*3). The day of administration was recorded as Day 0, second *in vivo* signal detection was performed on Day 6, subsequent signal detection was performed once every seven days, and mortality of mice in each group was recorded at the same time. The fluorescence signal detection image is shown in FIG. 1A, and the signal statistical results are shown in FIG. 1B. The results in FIG. 1 showed that, as time progressed, the *in vivo* signal in mice in the Vehicle group gradually increased, whereas although the in vivo signal in mice in the YSCH-01 group also increased slightly, the increase was significantly smaller than that in the Vehicle group. In this experiment, signal changes indicated the distribution and size of tumor cells in the mice, and therefore it could be concluded that, in a nude mouse A549 cell lung cancer model, YSCH-01 nebulized administration significantly inhibited the proliferation and spread of lung cancer and exhibited a good therapeutic effect.

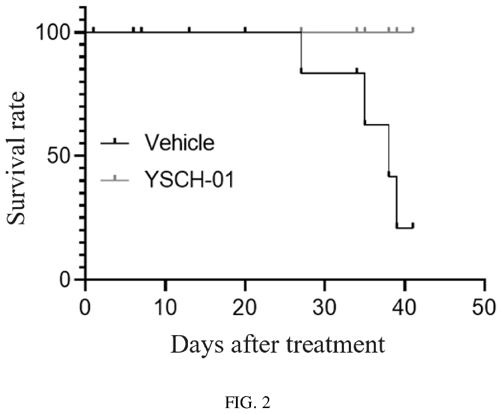

The survival curve of the mice is shown in FIG. 2, showing that no death occurred in mice in the YSCH-01 nebulized administration group from the first administration (Day 0) to 41 days after administration (Day 41), and the survival rate was 100%; whereas 4 mice died in the Vehicle group, and the survival rate was only 42.86%. Thus, YSCH-01 nebulized administration could significantly improve the survival rate of experimental mice with A549 orthotopic lung cancer.

### Example 3: Nebulization Therapy Using Oncolytic Virus for Lung Cancer Patient

### 1. Patient Conditions

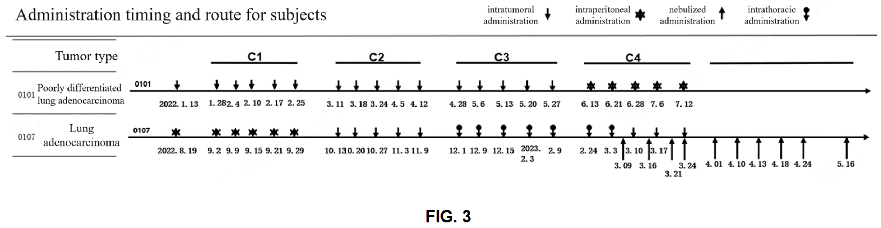

Patient 1, male, 65 years old, was diagnosed with left upper lobe lung adenocarcinoma T1bN3M1a stage IV, secondary malignant neoplasm of the lymph nodes, and secondary malignant neoplasm of the pleura. The administration time and administration mode for the patient are shown in FIG. 3. When the patient was in the low-dose group, the number was 0101; when the patient was in the high-dose group, the number was 0107.

### Low-dose group administration date:

January 13, January 28, February 4, February 10, February 17, and February 25, 2022 (first cycle)
March 11, March 18, March 24, April 5, and April 12 (second cycle).
April 28, May 6, May 13, May 20, and May 27 (third cycle).

In the first three cycles, puncture injection of recombinant L-IFN adenovirus injection was performed under ultrasound guidance at 5 x 10⁹ vp/ml, 1 ml, into the left supraclavicular lymph node. During administration, the patient's vital signs were stable, fever and the like did not occur, no adverse reaction occurred at the injection site, and no symptoms such as rash, dizziness, headache, joint pain, gastrointestinal reaction, subcutaneous hemorrhage, pruritus, palpitation, chest distress, nausea, vomiting, melena, and hematuria occurred, and the patient had a good mental status. During the three administration cycles, routine blood test, reticulocyte count, blood biochemistry, routine urine test, electrocardiogram, and the like were all within normal ranges. At the end of the third administration cycle, a second puncture pathological examination of the lymph node indicated that no malignant tumor cells were observed, B-mode ultrasound showed no suspected malignant lymph node in the superficial lymph nodes, and lung CT showed no obvious pleural effusion; after communication, the administration was changed to intraperitoneal administration from the fourth administration cycle.

The fourth cycle was performed on June 13, June 21, June 28, July 7, and July 12.

Puncture injection of recombinant L-IFN adenovirus injection at 5 × 10⁹ vp/ml, 1 ml, was performed at the right abdominal McBurney's point under ultrasound guidance. Administration in the low-dose group was completed.

After administration in the low-dose group was completed, the patient voluntarily requested to join the high-dose group, and the treatment was performed on:
August 19, September 2, September 9, September 15, September 21, and September 29, 2022 (first cycle).

Puncture injection of recombinant L-IFN adenovirus injection at 5 × 10¹¹ vp/ml, 1 ml, was performed at the right abdominal McBurney's point under ultrasound guidance, and follow-up routine blood test, reticulocyte count, blood biochemistry, routine urine test, electrocardiogram, and the like after administration were all within normal ranges.

The second cycle was performed on October 13, October 20, October 27, November 3, and November 9.

Puncture injection of recombinant L-IFN adenovirus injection at 5 x 10¹¹ vp/ml, 1 ml, into the left supraclavicular lymph node was performed under ultrasound guidance.

The third cycle was performed on December 1, December 9, December 15, December 22, 2022 and February 3, 2023.

The fourth cycle was performed on February 9, February 24, March 3, March 17, and March 24.

Thoracic cavity puncture injection of recombinant L-IFN adenovirus injection at 5 × 10¹¹ vp/ml, 1 ml, was performed under ultrasound guidance. During administration, the patient's vital signs were stable, fever and the like did not occur, no adverse reaction occurred at the injection site, and no symptoms such as rash, dizziness, headache, joint pain, gastrointestinal reaction, subcutaneous hemorrhage, pruritus, palpitation, chest distress, nausea, vomiting, melena, hematuria, abdominal pain, and abdominal distension occurred, and the patient had a good mental status. Routine blood test, reticulocyte count, blood biochemistry, routine urine test, electrocardiogram, and the like were all within normal ranges.

For the specific nebulized administration plan, see Table 5:

**Table 5**

| | | |
|---|---|---|
| 2023/3/9 | First oral inhalation nebulization dose | 3*10^9 vp/ml 1 ml, diluted with physiological saline to 5 ml |
| 2023/3/16 | Second oral inhalation nebulization dose | 5*10^10 vp/ml 1 ml, diluted with physiological saline to 5 ml |
| 2023/3/21 | Third oral inhalation nebulization dose | 5*10^10 vp/ml 1 ml, diluted with physiological saline to 5 ml |
| 2023/3/24 | Fourth oral inhalation nebulization dose | 2.5*10^11 vp/ml 1 ml, diluted with physiological saline to 5 ml |
| 2023/4/1 | Fifth oral inhalation nebulization dose | 3*10^11 vp/ml 1 ml, diluted with physiological saline to 5 ml |
| 2023/4/10 | Sixth oral inhalation nebulization dose | 3*10^11 vp/ml 1 ml, diluted with physiological saline to 5 ml |
| 2023/4/13 | Seventh oral inhalation nebulization dose | 5*10^11 vp/ml 1 ml, diluted with physiological saline to 5 ml |
| 2023/4/18 | Eighth oral inhalation nebulization dose | 5*10^11vp/ml 1 ml, diluted with physiological saline to 5 ml |
| 2023/4124 | Ninth oral inhalation nebulization dose | 5*10^11 vp/ml 1 ml, diluted with physiological saline to 5 ml |
| 2023/5/16 | Tenth oral inhalation nebulization dose | 5*10^11 vp/ml 1 ml, diluted with physiological saline to 5 ml |

For the detection results, see Table 6:

**Table 6**

| | Date | Color Doppler ultrasound of clavicular lymph node metastases | Plain CT scan of the thoracic primary lesion | Administration status | Puncture results |
|---|---|---|---|---|---|
| | 2021-12-02 | 20×15 mm | / | Before enrollment | |
| | 2021-12-24 | / | 8.3×9.9 mm | Before enrollment | |
| | 2021-12-31 | 17×9 mm | / | Before enrollment | |
| | 2022-01-08 | / | 21×12 mm | Before enrollment | |
| Intratumoral injection into cervical lymph nodes | 2022-01-13 | 14×8 mm | / | Low-dose C1, first injection | |
| | 2022-01-14 | 13×10 mm | / | | |
| | 2022-01-17 | 14×10 mm | / | | |
| | 2022-01-20 | 13×10 mm | / | | |
| | 2022-01-28 | 14×10 mm | / | | |
| | 2022-02-04 | 14×10 mm | Comparable to the previous film (21×12 mm) | | |
| | 2022-02-10 | 13 x 10 mm | / | | |
| | 2022-02-15 | 12 x 9 mm | / | | Muscle and adipose tissue |
| | 2022-02-17 | 11x7.8 mm | / | | |
| | 2022-02-25 | 12×8mm | | | |
| | 2022-03-11 | 12×8mm | 16×10mm | Low-dose C2, first injection | (left supraclavicular lymph node) lymph node metastatic lung adenocarcinoma (second lymph node) |
| | 2022-03-18 | 13×6mm | | | |
| | 2022-03-24 | 13×6mm | | | |
| | 2022-04-05 | 12×6mm | | | |
| | 2022-04-12 | 12×6mm | 16×14mm | | |
| | 2022-04-28 | 11×5mm | | Low-dose C3, first injection | |
| | 2022-05-06 | 11×5mm | | | |
| | 2022-05-13 | 11×5mm | | | |
| | 2022-05-20 | 11×5mm | | | |
| | 2022-05-27 | 11×5mm | | | (left supraclavicular lymph node) No malignant tumor cells were observed. (second lymph node). |
| Intraperitoneal injection administration | 2022-06-01 | | 17×12mm | Before C4 administration (intraperitoneal administration) | |
| | 2022-07-04 | | 16mm×9mm | | |
| | 2022-07-18 | 10×5×11mm | 16mm×9mm | End of C4 | B-mode ultrasound scanning of |
| | | | | administration | the left supraclavicular region showed several lymph nodes; (left supraclavicular lymph node puncture) no malignant tumor cells were found. |
| | 2022-08-18 | 10×7×11mm | 15mm×8mm | High-dose group before enrollment | |
| | 2022-08-24 | | | | 1. (cervical lymph node puncture) fibroadipose connective tissue. 2. (cervical lymph node puncture) no cancer metastasis was observed in the lymph node. 3. (cervical lymph node puncture) fibroadipose connective tissue. |
| Cervical lymph node injection | 2022-10-13 | 9.5×5.3mm | | High-dose C2, first injection | B-mode ultrasound showed several hypoechoic areas in the left supraclavicular region, with clear boundaries, and no obvious lymph hilum visualization. Blood flow signals were seen on CDFI. (suspected; subsequent puncture verified non-tumor tissue) |
| | 2022-10-20 | 9.7×5.4mm | 16mm×17mm | | B-mode ultrasound showed several hypoechoic areas with clear boundaries in the left supraclavicular region, and no obvious lymph hilum visualization. Blood flow signals were seen on CDFI. (suspected; subsequent puncture verified non-tumor tissue) |
| Intrathoracic injection administration | 2022-11-21 | | 16mm×17mm | Before the first injection of high-dose C3 | |
| | 2023-01-15 | | 14.8mm×16.1m m | | |
| Nebulized administration | 2023-03-18 | | 18mm×19mm | Second nebulized administration | |
| | 2023-04-10 | | 18mm×19mm | Sixth nebulized administration | |
| | 2023-05-06 | | 7.6mm×11.2mm | Ninth nebulized administration | |
| | 2023-05-23 | | | | B-mode ultrasound showed no obvious abnormalities in bilateral thyroid glands, and no obvious abnormal perithyroidal lymph nodes were observed; no obviously enlarged lymph nodes or lymph nodes with abnormal echogenicity were observed in bilateral supraclavicular regions; no obvious abnormal lymph nodes were observed in bilateral axillae; no obviously enlarged lymph nodes or lymph nodes with abnormal echogenicity were observed in bilateral inguinal regions. |

### Result evaluation:

Patient 1 had no obvious specific abnormalities in routine blood test and blood biochemistry before and after the two enrolled treatments.

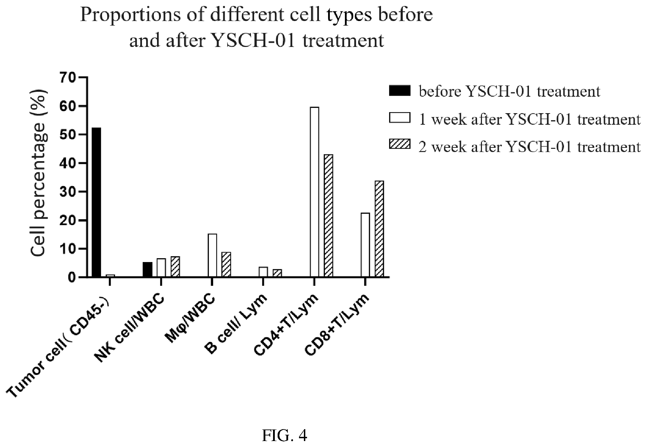

For survival follow-up, 15 months after enrollment (enrolled in January 2022), cervical puncture was still negative, and no recurrence was observed. During the treatment process, the second administration stage (C2) was evaluated as SD (Stable disease); subsequently, effusion occurred, the administration mode was adjusted to effusion drainage and administration, and the effusion was then significantly relieved (B-mode ultrasound of pleural effusion on November 23, 2022 showed that scanning of the left thoracic cavity revealed an anechoic area with a maximum depth of 70 mm below the seventh posterior rib. B-mode ultrasound of pleural effusion on March 3, 2023 showed a small amount of effusion in the left thoracic cavity, with the deepest portion being about 13 mm). After several treatments, the pleural effusion completely subsided. Immune cell testing was performed on the patient's pleural fluid before and after medication. Two analyses after administration showed that the immune cell populations were consistent with the trend that YSCH-01 activated immunity and reduced suppressiveness. After YSCH-01 administration, adherent CD45-negative cells (mainly tumor cells) decreased substantially, and pathological examination showed no viable tumor cells. For the detection results of immune cell population proportions before and after oncolytic virus treatment, see FIG. 4.

Bone metastasis subsequently occurred, and the patient withdrew from the study due to disease progression. Follow-up after withdrawal from the study showed remission in the condition of Patient 1. Patient 1 requested continued administration. On March 24, 2023, the fifth dose of C4 was started. Positron emission tomography-computed tomography system (PET-CT) examination results showed that the standardized uptake value (SUV) of tumor tissue was significantly reduced to <5, indicating reduced tumor activity after nebulized administration. The primary lung lesion decreased from the original 18 mm x 19 mm (measured on March 18, 2023) to 7.6 mm x 11.2 mm (measured on May 6, 2023), indicating that nebulized administration of an oncolytic virus could control progression of lung tumor disease and rapidly mobilize mucosal immunity to kill tumor cells; the tumor size of the primary lung lesion was significantly reduced, the prognosis of nebulization therapy was good, and unexpected therapeutic effects were achieved.

The above descriptions are only some embodiments of the present disclosure and are not intended to limit the scope of protection of the present disclosure. Any modifications, equivalent replacements, improvements, and the like made within the spirit and principles of the present disclosure are all included within the scope of protection of the present disclosure.

## Claims

1. Use of an oncolytic virus in the manufacture of an aerosol drug for treating lung cancer, wherein the oncolytic virus is an interferon-expressing oncolytic virus.

2. The use according to claim 1, wherein the interferon is interferon α, interferon β, interferon γ, or consensus interferon.

3. The use according to claim 1 or 2, wherein the interferon is interferon α, preferably the interferon is consensus interferon, more preferably the interferon is encoded by a nucleotide sequence of SEQ ID NO: 1, SEQ ID NO: 2, or SEQ ID NO: 3.

4. The use according to any one of claims 1 to 3, wherein the oncolytic virus is an attenuated wild-type virus strain, such as a reovirus and a Newcastle disease virus; alternatively, the oncolytic virus is a genetically engineered virus, such as an engineered adenovirus, a herpes simplex virus, a vaccinia virus, and a measles virus.

5. The use according to any one of claims 1 to 4, wherein the oncolytic virus is an oncolytic adenovirus.

6. The use according to any one of claims 1 to 5, wherein the aerosol drug is administered by a metered dose inhaler, a dry powder inhaler, and/or a nebulizer, preferably a nebulizer.

7. The use according to claim 6, wherein the nebulizer is selected from the group consisting of a pneumatic jet nebulizer, an ultrasonic nebulizer, and a mesh nebulizer.

8. The use according to any one of claims 1 to 7, wherein the lung cancer is small cell lung cancer, lung squamous cell carcinoma, lung adenocarcinoma, large cell lung cancer, and/or metastatic lung tumor.

9. An oncolytic virus for treating lung cancer, wherein the oncolytic virus is administered in an aerosol form and the oncolytic virus is an interferon-expressing oncolytic virus.

10. The oncolytic virus according to claim 9, wherein the interferon is interferon α, interferon β, interferon γ, or consensus interferon.

11. The oncolytic virus according to claim 9 or 10, wherein the interferon is interferon α, preferably the interferon is consensus interferon, more preferably the interferon is encoded by a nucleotide sequence of SEQ ID NO: 1, SEQ ID NO: 2, or SEQ ID NO: 3.

12. The oncolytic virus according to any one of claims 9 to 11, wherein the oncolytic virus is an attenuated wild-type virus strain, such as a reovirus and a Newcastle disease virus; alternatively, the oncolytic virus is a genetically engineered virus, such as an engineered adenovirus, a herpes simplex virus, a vaccinia virus, and a measles virus.

13. The oncolytic virus according to any one of claims 9 to 12, wherein the oncolytic virus is an oncolytic adenovirus.

14. The oncolytic virus according to any one of claims 9 to 13, wherein the aerosol form is administered by a nebulization device, and the nebulization device is selected from the group consisting of a metered dose inhaler, a dry powder inhaler, and a nebulizer, preferably a nebulizer.

15. The oncolytic virus according to claim 14, wherein the nebulizer is selected from the group consisting of a pneumatic jet nebulizer, an ultrasonic nebulizer, and a mesh nebulizer.

16. The oncolytic virus according to any one of claims 9 to 15, wherein the lung cancer is small cell lung cancer, lung squamous cell carcinoma, lung adenocarcinoma, large cell lung cancer, and/or metastatic lung tumor.

17. A method for treating lung cancer, comprising administering an oncolytic virus to a subject in need thereof via nebulization, wherein the oncolytic virus is an interferon-expressing oncolytic virus.

18. The method according to claim 17, wherein the interferon is interferon α, interferon β, interferon γ, or consensus interferon.

19. The method according to claim 17 or 18, wherein the interferon is interferon α, preferably the interferon is consensus interferon, more preferably the interferon is encoded by a nucleotide sequence of SEQ ID NO: 1, SEQ ID NO: 2, or SEQ ID NO: 3.

20. The method according to any one of claims 17 to 19, wherein the oncolytic virus is an attenuated wild-type virus strain, such as a reovirus and a Newcastle disease virus; alternatively, the oncolytic virus is a genetically engineered virus, such as an engineered adenovirus, a herpes simplex virus, a vaccinia virus, and a measles virus.

21. The method according to any one of claims 17 to 20, wherein the oncolytic virus is an oncolytic adenovirus.

22. The method according to any one of claims 17 to 21, wherein the nebulization is performed by a nebulization device, and the nebulization device is selected from the group consisting of a metered dose inhaler, a dry powder inhaler, and a nebulizer, preferably a nebulizer.

23. The method according to claim 22, wherein the nebulizer is selected from the group consisting of a pneumatic jet nebulizer, an ultrasonic nebulizer, and a mesh nebulizer.

24. The method according to any one of claims 17 to 23, wherein the lung cancer is small cell lung cancer, lung squamous cell carcinoma, lung adenocarcinoma, large cell lung cancer, and/or metastatic lung tumor.

25. The method according to any one of claims 17 to 24, further comprising administering the oncolytic virus to the subject by intratumoral injection and/or intraperitoneal injection.

26. The method according to any one of claims 17 to 25, further comprising administering an antitumor drug to the subject, wherein the antitumor drug is selected from the group consisting of a chemotherapeutic drug, such as paclitaxel, cisplatin, carboplatin, pemetrexed, docetaxel, gemcitabine, and irinotecan, a targeted therapeutic drug, such as gefitinib, osimertinib, erlotinib, afatinib, and lapatinib, and an immunotherapeutic drug, such as bevacizumab, nimotuzumab, sintilimab, camrelizumab, nivolumab, pembrolizumab, atezolizumab, durvalumab, and ipilimumab.

27. The method according to any one of claims 17 to 26, further comprising performing radiotherapy on the subject.

28. An aerosol pharmaceutical composition, comprising an oncolytic virus, wherein the aerosol pharmaceutical composition is in a liquid form or an aerosol form.

29. The aerosol pharmaceutical composition according to claim 28, wherein the oncolytic virus is an interferon-expressing oncolytic virus, wherein the interferon is interferon α, interferon β, interferon γ, or consensus interferon.

30. The aerosol pharmaceutical composition according to claim 28 or 29, wherein the interferon is interferon α, preferably the interferon is consensus interferon, more preferably the interferon is encoded by a nucleotide sequence of SEQ ID NO: 1, SEQ ID NO: 2, or SEQ ID NO: 3.

31. The aerosol pharmaceutical composition according to any one of claims 28 to 30, wherein the aerosol pharmaceutical composition is administered by a metered dose inhaler, a dry powder inhaler, and/or a nebulizer, preferably a nebulizer, such as a pneumatic jet nebulizer, an ultrasonic nebulizer, and a mesh nebulizer.

32. An aerosol kit, comprising a nebulization device and a drug, wherein the drug comprises an oncolytic virus and the drug is in a liquid form or an aerosol form.

33. The aerosol kit according to claim 32, wherein the oncolytic virus is an interferon-expressing oncolytic virus, wherein the interferon is interferon α, interferon β, interferon γ, or consensus interferon.

34. The aerosol kit according to claim 32 or 33, wherein the interferon is interferon α, preferably the interferon is consensus interferon, more preferably the interferon is encoded by a nucleotide sequence of SEQ ID NO: 1, SEQ ID NO: 2, or SEQ ID NO: 3.

35. The aerosol kit according to any one of claims 32 to 34, wherein the nebulization device is selected from the group consisting of a metered dose inhaler, a dry powder inhaler, and a nebulizer, preferably a nebulizer, such as a pneumatic jet nebulizer, an ultrasonic nebulizer, and a mesh nebulizer.
